# EUROPEAN PATENT APPLICATION

(11) **EP 3 806 106 A1**
(43) Date of publication of application: **14.04.2021**
(21) Application number: 20150465.1
(22) Date of filing: 07.01.2020
(51) Int. Cl.: G16H 50/00, G16H 50/20

(54) **SCALABLE CLINICAL DECISION SUPPORT**

(30) Priority: 10.10.2019 US 201962913384 P
(71) Applicant: Koninklijke Philips N.V., 5656 AG Eindhoven (NL)
(72) Inventor: VAN ZON, Cornelis Conradus Adrianus Maria, 5656 AE Eindhoven (NL)
(74) Representative: Steenbeek, Leonardus Johannes

(57) **Abstract**

A system and method for providing information supportive of clinical decision making. The method includes receiving (212) a primary set of input data, and producing (214) a first output having a first performance value based upon the primary set of input data. The method produces (220) a second output having a second performance value based upon the primary set of input data and a supplemental set of input data, when the supplemental set of input data is available and the second performance value of the second output exceeds the first performance value of the first output.

## Description

### TECHNICAL FIELD

This invention generally relates to systems and methods for algorithmic clinical decision support and, more particularly but not exclusively, to systems and methods for providing information for clinical decisions using data sources with mixed availability.

### BACKGROUND

Clinical decision support (CDS) refers to computer-based support to clinical decision making. Generally, CDS systems provide one or more of clinicians, staff, and patients with medical knowledge or information relevant to an individual patient and/or context. The CDS system takes as input patient-specific data of certain types. Examples of types of patient specific data types include: demographics, health history, family history, allergies, medication use, past procedures, signs and symptoms, test results (e.g., labs and radiology), clinician notes, and vitals. CDS has a number of demonstrated benefits including increased quality of care for the patient; improved avoidance of medical errors; and improved clinical efficiency.

Presently, the number of and interoperability between medical devices in a typical clinic or hospital is growing. This growing number of medical devices are generating more and more patient specific information of many different types available at the time of clinical decision making. However, current CDS systems are often developed to consider only a defined set of patient-specific information types and less than all of the available patient specific data is being used as input to providing clinical decision support.

### SUMMARY

Improved systems and methods for CDS are needed to make use of the varying availability and large amounts of patient information types currently available. The invention is defined by the independent claims. The dependent claims define advantageous embodiments.

This summary is provided to introduce a selection of concepts in a simplified form that are further described below in the Detailed Description section. This summary is not intended to identify or exclude key features or essential features of the claimed subject matter, nor is it intended to be used as an aid in determining the scope of the claimed subject matter.

In one aspect, embodiments relate to a method for providing information supportive of clinical decision making. The method using a processor includes receiving a primary set of input data; producing a first output having a first performance value based upon the primary set of input data; receiving a signal indicating an availability of a supplemental set of input data; receiving the supplemental set of input data when available; and, producing a second output having a second performance value based upon the primary set of input data and the supplemental set of input data when the supplemental set of input data is available, wherein the second performance value exceeds the first performance value.

Embodiments of the invention are based on the recognition that the increasing interoperability among medical devices, electronic medical records (EMRs) and data analytics platforms offers opportunities for a new CDS paradigm: algorithms or solutions that have a minimum set of input data that is mandatory to warrant a particular performance, but that can make use of certain additional data, if and when that is available, to increase their performance if by doing so, the performance is indeed increased. The result is a scalable clinical decision support (SCDS). It depends on the exact function of an algorithm what performance means; that can be prediction accuracy, confidence in recommendations, relevance of contextual data, etc.

In some embodiments of the method, the method using the processor additionally includes receiving a second signal indicating the availability of a second supplemental set of input data; and producing a third output having a third performance value based upon the primary set of input data and the second supplemental set of input data, when the second supplemental set of input data is available and the third performance value exceeds the first performance value. The second supplemental set of input data may or may not include the supplemental set of input data.

In an example, the supplemental set of input data includes oxygen saturation, and the second performance value falls below the first performance value so that the oxygen saturation alone is not used to produce the second output. However, the second supplemental set of input data includes both oxygen saturation and age, and the third performance value exceeds the first performance value, so that the combination of oxygen saturation and age is used to produce the third output.

In another example,
- the supplemental set of input data includes oxygen saturation, and the second performance value falls below the first performance value so that the oxygen saturation alone is not used to produce the second output;
- the second supplemental set of input data includes both oxygen saturation and age, and the third performance value too falls below the first performance value, so that the combination of oxygen saturation and age is not used to produce the third output; and
- a third supplemental set of input data includes age without oxygen saturation, and the corresponding performance value exceeds the first performance value, so that the age is used to produce an enhanced output that improves the first output.

In some embodiments of the method, the method additionally includes receiving, at the processor, information concerning the contents of the primary set of input data and the supplementary set of input data.

In some embodiments of the method, the method additionally includes providing, using the processor, a confidence value associated with the first output or the second output.

In some embodiments of the method, the method additionally includes indicating, using the processor, an absence of the primary set of input data when the primary set of input data is at least partially incomplete. In some cases, indicating the absence of the primary set of input data includes at least one of: logging the absence of the primary set of input data in an electronic medical record; mentioning the absence of the primary set of input data in a report; flagging the absence of the primary set of input data on a user interface; and providing an alarm indicating the absence of the primary set of input data.

In some embodiments of the method, the method additionally includes storing, using the processor, at least one of the primary set of input data, the supplemental set of input data, the first output, and the second output in a log file.

In some embodiments of the method, the signal indicating the availability of the supplemental set of input data is provided by means of a publish/subscribe mechanism.

In some embodiments of the method, the first output includes a receiver operating characteristics (ROC) curve.

In some embodiments of the method, the first performance value comprises at least one of an area under the curve (AUC) calculation and a confidence calculation.

In some embodiments, the method uses a list of supplemental input data types and corresponding performance values, so that when certain supplemental input data is available, a quick decision can be reached as to whether to (receive and) use certain supplemental input data types. The list may be updated each time the CDS algorithm is updated.

In another aspect, embodiments relate to a system for providing information supportive of clinical decision making. The system includes a processor configured to receive a primary set of input data; produce a first output having a first performance value based upon the primary set of input data; receive a signal indicating an availability of a supplemental set of input data; receive the supplemental set of input data when it is available; and, produce a second output having a second performance value based upon the primary set of input data and the supplemental set of input data when the supplemental set of input data is available, wherein the second performance value exceeds the first performance value.

In some embodiments of the system, the processor is further configured to receive a second supplemental set of input data when it is available; and, produce a third output having a third performance value based upon the primary set of input data and at least one of the supplemental set of input data, when the supplemental set of input data is available, and the second supplemental set of input data, when the second supplemental set of input data is available.

In some embodiments of the system, the processor is additionally configured to receive information concerning the contents of the primary set of input data and the secondary set of input data.

In some embodiments of the system, the processor is additionally configured to provide a confidence value association with the first output or the second output.

In some embodiments of the system, the processor is additionally configured to provide an alarm when the primary set of input data is at least partially incomplete.

In some embodiments of the system, the processor is additionally configured to store at least one of the primary set of input data, the supplemental set of input data, the first output, and the second output in a log file.

In some embodiments of the system the signal indicating the availability of the supplemental set of input data is a publish/subscribe mechanism.

In some embodiments of the system, the first output includes a receiver operating characteristics (ROC) curve.

In some embodiments of the system, the first performance value comprises at least one of an area under the curve (AUC) calculation and a confidence calculation.

In yet another aspect, some embodiments relate to a non-transitory computer readable media storing instructions that are executable by a processing device. Upon execution of the instructions, the processing device performs operations that include receiving a primary set of input data; producing a first output having a first performance value based upon the primary set of input data; receiving a signal indicating an availability of a supplemental set of input data; receiving the supplemental set of input data when available; and, producing a second output having a second performance value based upon the primary set of input data and the supplemental set of input data when the supplemental set of input data is available, wherein the second performance value exceeds the first performance value.

In some embodiments of the non-transitory computer readable media storing instructions that are executable by a processing device, the operations additionally include receiving information concerning the contents of the primary set of input data and the supplementary set of input data.

Any combination and permutation of embodiments is envisioned. Other objects and features will become apparent from the following detailed description considered in conjunction with the accompanying drawings. It is to be understood, however, that the drawings are designed as an illustration only and not as a definition of the limits of the present disclosure.

### BRIEF DESCRIPTION OF DRAWINGS

Non-limiting and non-exhaustive embodiments of the invention are described with reference to the following figures, wherein like reference numerals refer to like parts throughout the various views unless otherwise specified.
FIG. 1 illustrates an exemplary scalable clinical decision support (SCDS) system in accordance with one embodiment;
FIG. 2A presents a flowchart of an exemplary method for providing information in support of a clinical decision based upon a primary set of input data and a supplemental set of input data performed in accordance with one embodiment;
FIG. 2B presents a flowchart of a method for providing clinical decision support information in response to changes in availability of patient data in accordance with one embodiment;
FIG. 2C presents a flowchart of a method for recognizing changes in availability of patient data in accordance with one embodiment;
FIG. 3 depicts an exemplary system for providing information in support of a clinical decision based upon a primary and a supplemental set of input data performed in accordance with one embodiment;
FIG. 4A shows a first receiver operating characteristic (ROC) curve according to an exemplary embodiment; and,
FIG. 4B shows a second receiver operating characteristic (ROC) curve according to an exemplary embodiment.

### DETAILED DESCRIPTION OF EMBODIMENTS

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment.

Some portions of the description that follow are presented in terms of symbolic representations of operations on non-transient signals stored within a computer memory. These descriptions and representations are used by those skilled in the data processing arts to most effectively convey the substance of their work to others skilled in the art. Such operations typically require physical manipulations of physical quantities. Usually, though not necessarily, these quantities take the form of electrical, magnetic or optical signals capable of being stored, transferred, combined, compared and otherwise manipulated. It is convenient at times, principally for reasons of common usage, to refer to these signals as bits, values, elements, symbols, characters, terms, numbers, or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems.

The present disclosure also relates to an apparatus for performing the operations herein. This apparatus may be specially constructed for the required purposes, such as a distributed system involving multiple computers or servers (physical and/or virtual), or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

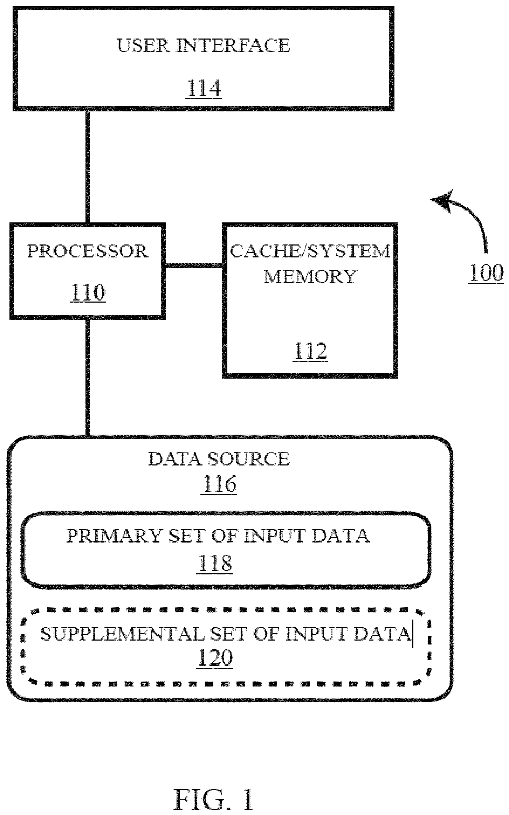

FIG. 1 illustrates a scalable clinical decision support (SCDS) system 100 according to some embodiments. The SCDS system 100 provides information that is supportive of clinical decisions based upon available data. The SCDS system 100 shown in FIG. 1 includes a processor 110, and a system memory 112. The system memory 112 in some embodiments comprises at least one of static random-access memory (SRAM), dynamic RAM (DRAM), flash memory, read only memory (ROM), or other similar memory devices and configurations. The processor 110 is configured to provide information to support clinical decisions. In some embodiments, the system 100 includes a user interface 114 and the information to support clinical decisions is provided to the user interface. In some embodiments, the user interface 114 is local to the processor 110. Alternatively, the user interface in some embodiments is remote from the processor 110 and the information supportive of clinical decisions is communicated to the user interface by way of one or more networks (e.g., LAN, WAN, WiFi, Bluetooth, etc.). The SCDS 100 is shown in communication with a data source 116. The data source 116 can be said to comprise a primary set of input data 118 and at least one supplemental set of input data 120. The data source 116 in some embodiments comprises patient information. The input data can be data live streaming from medical devices such as patient monitors and mechanical ventilators, and/or recorded data requested from sources such as the electronic medical record system, the lab system, etcetera.

Patient information includes dynamic patient information and non-dynamic patient information. Examples of non-dynamic patient information are patient characteristics that generally do not change (or change predictably), such as: age (date of birth), race, sex, occupation, blood type, maintenance medications the patient has been taking, etc. Examples of dynamic patient information are patient characteristics that can change, such as: pulse, oxygen saturation level, blood pressure, deterioration rate, etc. In some embodiments, the data source 116 comprises multiple sub-systems, where one sub-system provides non-dynamic patient data and another sub-system provides dynamic patient data. An example of a sub-system that includes non-dynamic patient data is a patient information database. An example of a sub-system that includes dynamic patient data is an electrocardiogram, which continually monitors electrical signals in a patient's heart.

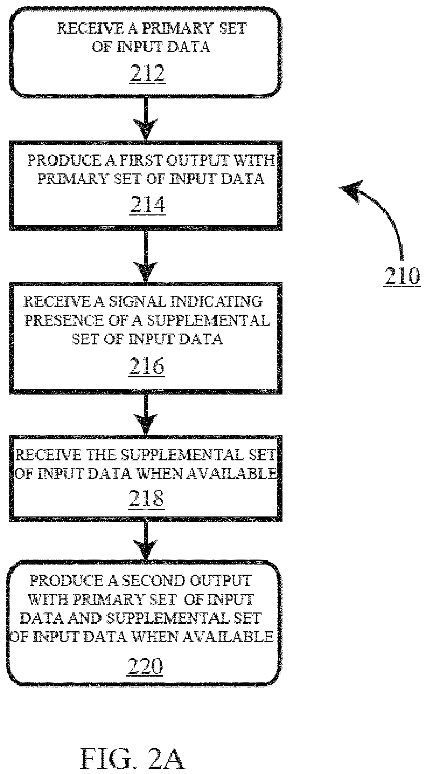

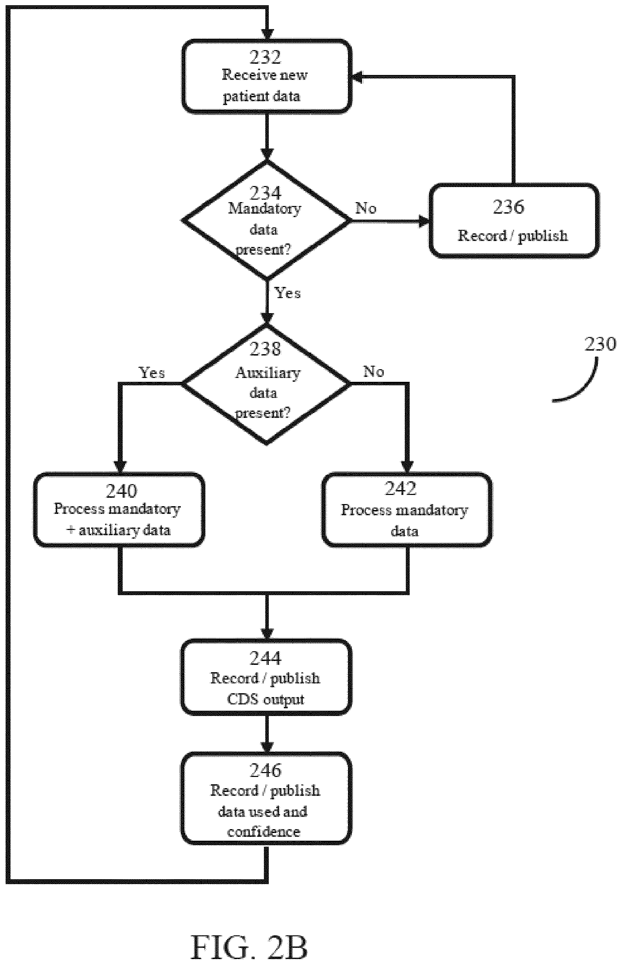

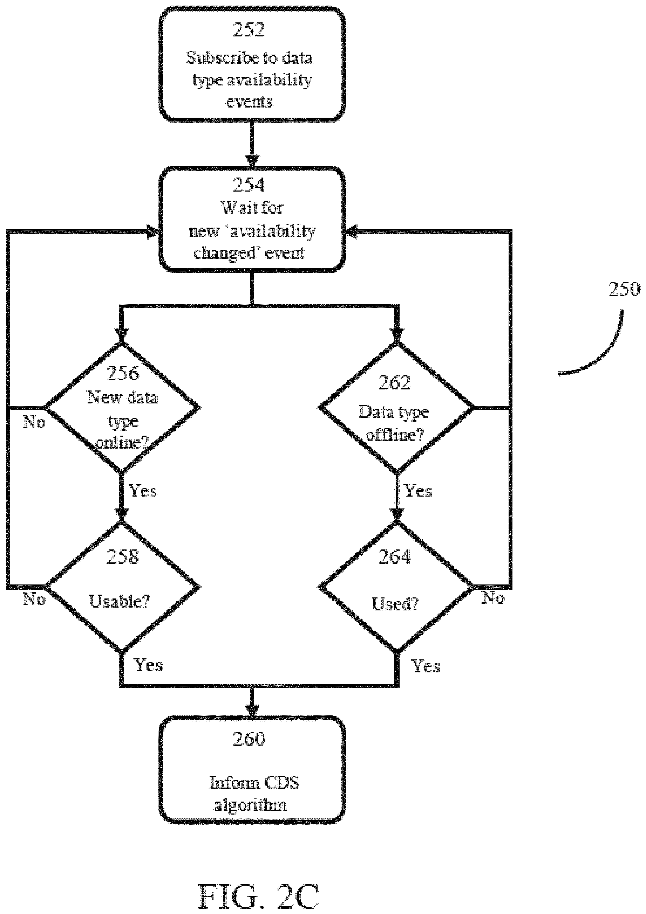

Operation of an SCDS method 210, according to some embodiments is represented by a flow chart in FIG. 2. First, a primary set of input data is received 212. According to some embodiments, the primary set of input data comprises patient specific information that is used by the SCDS system 100 to generate a meaningful output. For example, the primary set of input data may comprise one or more patient information types that are known to be required for an algorithmic assessment.

Then, the primary set of input data is used to produce a first output 214. Typically, one or more algorithms are employed to produce the first output. The first output in some embodiments represents one or more of a statistical classification (e.g., determination of an index or factor); a binary classification (e.g., an identification of a categorization); an inference; and a calculated value (e.g., an organ status score). In some cases, the first output is provided from inference of medical knowledge and the primary set of input data. In some embodiments, the first output has a performance value. In some cases, the performance value is a representation of one of accuracy of the first output and precision of the first output. For example, in some embodiments, the first performance value represents area under the curve (AUC) for a receiver operating characteristic curve. Typically, the first output has a performance value that is no less than a minimum acceptable value. This minimum acceptable value is a predetermined threshold below which the SCDS will not provide output.

Next, the method 210 receives a signal indicating presence of a supplemental set of input data 216. In some embodiments, the signal indicating presence of a supplemental set of input data is communicated by way of a publish and subscribe system.

The next step of the method 210 is to receive the supplemental set of input data when available 216. The supplemental set of input data in some embodiments, is received from one or more of a medical device, an electronic health record (EHR), and an electronic medical record (EMR). For example, medical devices that can provide the supplemental set of input data in some embodiments include: Magnetic Resonance Imaging (MRI) system, Electrocardiogram (ECG) system, heart rate monitor, pulse oximetry system, echocardiogram, ultrasound imaging systems, etc. An exemplary EMR is Tasy EMR from Koninklijke Philips N.V.

Finally, the next step of the method 210 is to produce a second output with a primary set of input data and a supplementary set of input data when available 220. Typically, one or more algorithms are employed to produce the second output. The second output in some embodiments represents one or more of a statistical classification (e.g., determination of an index or factor); a binary classification (e.g., an identification of a categorization); an inference; and a calculated value. In some cases, the second output is provided from inference of medical knowledge and the primary set of input data. According to some embodiments, the second output has a second performance value. In some cases, the second performance value is a representation of one of accuracy of the second output and precision of the second output. In some cases, the second performance value is greater than the first performance value of the first output 214. Where the second performance value is greater than the first performance value, the second output is deemed to be more useful for clinical decision making than the first output.

FIG. 2B shows a second flow chart 230 to illustrate the logic of an exemplary SCDS method being executed by an SCDS system 100. The flow chart 230 begins with the SCDS system 100 receiving new patient data 232. As described above, new patient data may be received from many sources (e.g., connected medical devices, electronic medical records, etc.). The new patient data is then scrutinized to determine if it contains mandatory data 234. The mandatory data is a minimum set of input data required to provide an output (e.g., a primary set of input data). If it is determined that the new patient data does not contain the mandatory data, the present absence of mandatory data is reported 236. According to some embodiments, reporting on the absence of mandatory data 236 is done by one or more of a publishing process and a recording process. For example, the SCDS is some cases publishes to a user interface and records to a log file that the mandatory patient data is not present in the new patient data. After reporting on the absence of mandatory data in the new patient data the SCDS 100 attempts again to receive new patient data 232.

If after receiving the new patient data, the SCDS 100 determines the mandatory data to be present in the patient data, the SCDS 100 further determines if auxiliary data is present 238. Auxiliary data represents information that the SCDS 100 can use to improve its output, but which does not need to be used in calculating an output (e.g., a supplemental set of input data). If auxiliary data is found within the patient data, both the mandatory data and the auxiliary data are processed 240 by the SCDS 100. If instead the auxiliary data is absent from the patient data, only the mandatory data is processed 242 by the SCDS 100. Next, the SCDS 100 reports an output of the processing 244. Finally, the SCDS 100 reports the confidence (e.g., performance value) of the output 246. In some embodiments, the SCDS 100 publishes and/or records the output and the confidence through systems and methods described above.

FIG. 2C shows a flowchart 250 that represents how an SCDS 100 receives new patient data, according to some embodiments. First the SCDS 100 subscribes to data type availability events 252. Then the SCDS 100 waits for a new 'availability changed' event 254. An example of an 'availability changed' event can include patient data being made available and patient data being made unavailable. The SCDS 100 tests to determine if a new data type is available 256. If the new data is available, the SCDS 100 then tests to determine if the new data type is usable 258. If the new data type is both online and available, the SCDS 100 informs the CDS algorithm 260. The SCDS upon a new availability change event in some embodiments determines if a data type has gone offline 262. If the data type has gone offline the SCDS 100 determines if the offline data type is used by the CDS algorithm 264. If the data type has both gone offline and is used by the CDS algorithm, the CDS algorithm is informed 260.

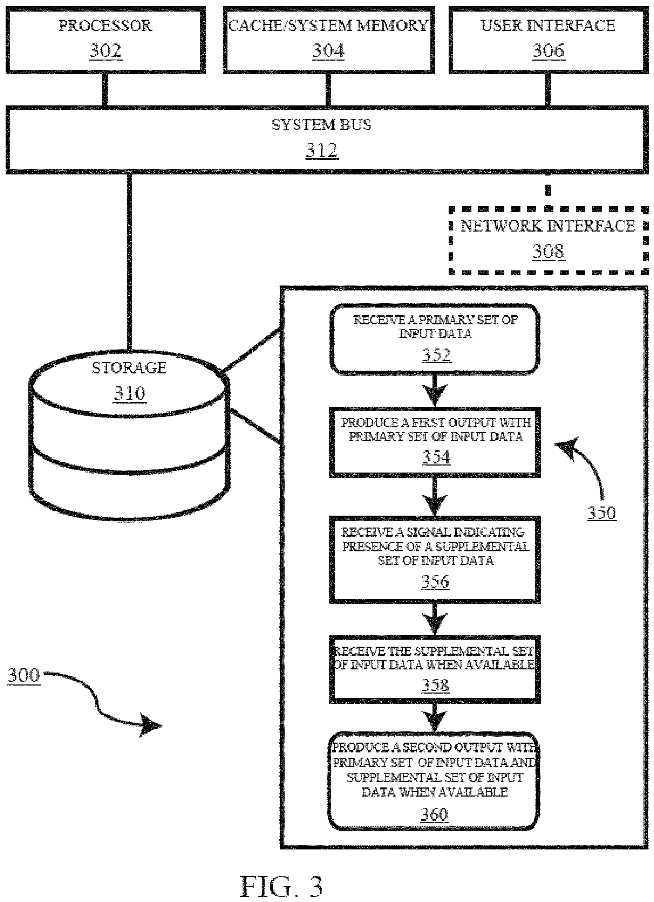

Referring now to FIG. 3, a system for scalable clinical decision making 300 in accordance with one embodiment is shown. The system 300 may include a processor 302, memory 304, a user interface 306, a network interface 308, and storage 310, all interconnected via one or more system buses 312. It will be understood that FIG. 3 constitutes, in some respects, an abstraction and that the actual organization of the system 300 and the components thereof may differ from what is illustrated.

The processor 302 may be any hardware device capable of executing instructions stored on memory 304 and/or in storage 310, or otherwise any hardware device capable of processing data. As such, the processor 302 may include a microprocessor, field programmable gate array (FPGA), an application-specific integrated circuit (ASIC), or other similar devices.

The memory 304 may include various transient memories such as, for example L1, L2, or L3 cache or system memory. As such, the memory 304 may include static random-access memory (SRAM), dynamic RAM (DRAM), flash memory, read only memory (ROM), or other similar memory devices and configurations.

The user interface 306 may include one or more devices for enabling communication with system operators and other personnel. For example, the user interface 306 may include a display, a mouse, and a keyboard for receiving user commands. In some embodiments, the user interface 306 may include a command line interface or graphical user interface that may be presented to a remote terminal via the network interface 306. The user interface 306 may execute on a user device such as a PC, laptop, tablet, mobile device, or the like, and may enable a user to provide the system 300 with a plurality of search request. Additionally, one or more user interfaces 306 may be used to report results of a clinical decision support system 300.

The network interface 308 may include one or more devices for enabling communication with other remote devices to access patient data. For example, the network interface 308 may include a network interface card (NIC) configured to communicate according to the Ethernet protocol. Additionally, the network interface 308 may implement a TCP/IP stack for communication according to the TCP/IP protocols. The system 300 may access patient data by way of the network interface 308, for example, through interconnected medical devices and/or networked electronic health records. Additionally, reporting of clinical decision support results 244 and confidence 246 may be performed via the network interface 308. Various alternative or additional hardware or configurations for the network interface 308 will be apparent.

The storage 310 may include one or more machine-readable storage media such as read-only memory (ROM), random-access memory (RAM), magnetic disk storage media, optical storage media, flash-memory devices, or similar storage media. In various embodiments, the storage 310 may store instructions for execution by the processor 302 or data upon which the processor 302 may operate. For example, the storage 310 may include instructions to: receive a primary set of input data 352; produce a first output with the primary set of input data 354; receive a signal indicating presence of a supplemental set of input data 356; receive the supplemental set of input data when available 358; and, produce a second output with primary set of input data and supplemental set of input data when available 360.

According to some embodiments, the instructions stored on the one or more machine-readable storage media additionally include receiving information concerning the contents of the primary set of input data and the supplemental set of input data.

The present disclosure is further explained below in reference to an example scalable clinical decision support (SCDS). The example SCDS uses an early deterioration index (EDI) algorithm in order to attempt to predict a likelihood that a patient's health will precipitously deteriorate within a certain timeframe. The example SCDS takes as inputs following patient data: pulse rate (PR), deterioration rate (RR), age, and oxygen saturation (SpO2).

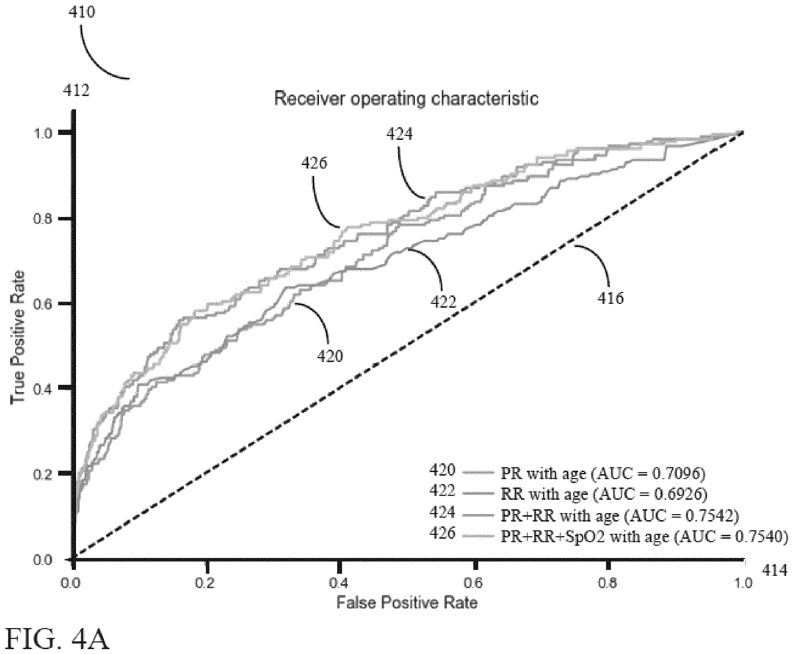

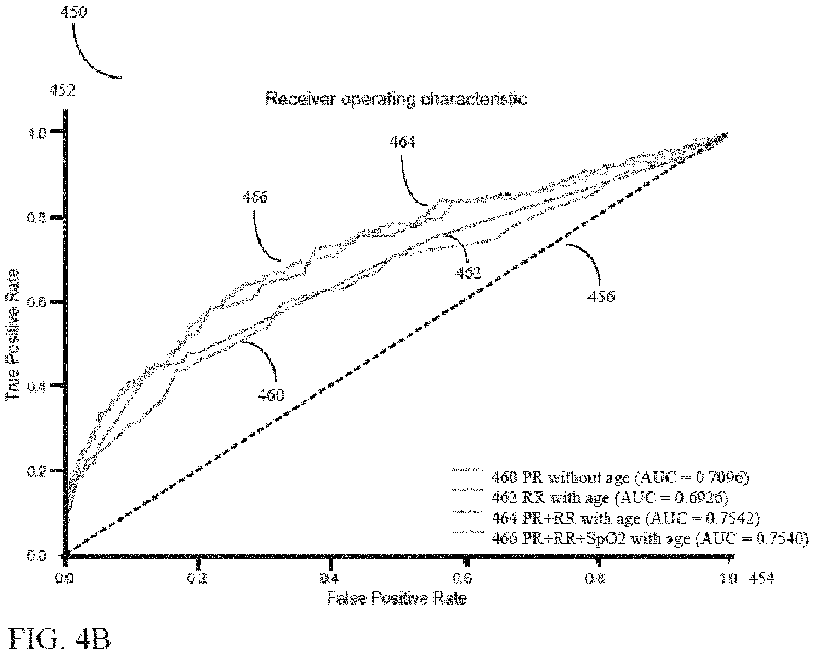

The example SCDS receives new values for pulse rate, deterioration rate, and oxygen saturation every second. FIGS. 4A and 4B show receiver operating characteristic (ROC) curves and area under the curve (AUC) values for the example SCDS. FIG. 4A shows an ROC plot and AUC values with the example SCDS including patient age in its determination. And, FIG. 4B shows an ROC plot and AUC values with the example SCDS omitting patient age in its determination.

Referring to FIG. 4A, a receiver operating characteristics (ROC) curve is shown in a first plot 410. The first plot shows performance of the example SCDS when age is included in determining EDI. The first plot 410 represents sensitivity (e.g., probability of detection or true positive rate) along a vertical axis 412. Specificity (e.g., 1-sensitivity, or probability of false alarm, or false positive rate) is represented along a horizontal axis 414. A line of non-discrimination 416 cuts diagonally across the first plot 410. A receiver operating characteristic (ROC) curve illustrates the ability of a classification system as its discrimination threshold is varied. The line of non-discrimination 416 represents a threshold, below which a classification system can be said to be non-discriminating (e.g., no better than random). Four cumulative distribution functions (CDFs) are plotted on the first plot 410. Each point of each CDF function represents results from an individual confusion matrix in cartesian form, for example where X = true positive rate (TPR) and Y = false positive rate (FPR) (e.g., (TPR, FPR)). The four CDF functions were calculated each with different data types available to the example SCDS. For example, a PR with age CDF 420 includes pulse rate and age; a RR with age CDF 422 includes deterioration rate and age; PR + RR with age CDF 424 includes pulse rate and deterioration rate and age; and, a PR + RR + SpO2 CDF with age 426 includes pulse rate, deterioration rate, oxygen saturation, and age. An area under the curve (AUC) calculation is provided for each CDF. The AUC calculation represents relative performance of classification of the CDF. The PR with age CDF 420 has an AUC of 0.7096; the RR with age CDF 422 has an AUC of 0.6926; The PR + RR with age CDF 424 has an AUC of 0.7542; and the PR+ RR+ SpO2 with age CDF 426 has an AUC of 0.7540.

Referring to FIG. 4B, a receiver operating characteristics (ROC) curve is shown in a second plot 450. The second plot shows 450 performance of the example SCDS when patient age is omitted in determining EDI. The second plot 450 represents sensitivity (e.g., probability of detection or true positive rate) along a vertical axis 452. Specificity (e.g., 1-sensitivity, or probability of false alarm, or false positive rate) is represented along a horizontal axis 454. A line of non-discrimination 456 cuts diagonally across the second plot 450. A receiver operating characteristic (ROC) curve illustrates the ability of a classification system as its discrimination threshold is varied. The line of non-discrimination 456 represents a threshold, below which a classification system can be said to be non-discriminating (e.g., no better than random). Four cumulative distribution functions (CDFs) are plotted on the second plot 450. Each point of each CDF function represents results from an individual confusion matrix in cartesian form, where for example X = true positive rate (TPR) and Y = false positive rate (FPR) (e.g., (TPR, FPR)). The four CDF functions were calculated each with different data types available to the example SCDS. For example, a PR without age CDF 460 includes pulse rate and omits age; a RR without age CDF 462 includes deterioration rate and omits age; PR + RR without age CDF 464 includes heart rate and deterioration rate and omits age; and, a PR + RR + SpO2 CDF without age 466 includes heat rate, deterioration rate, oxygen saturation, and omits age. An area under the curve (AUC) calculation is provided for each CDF. The AUC calculation represents relative performance of classification of the CDF. The PR without age CDF 460 has an AUC of 0.6524; the RR without age CDF 462 has an AUC of 0.6698; The PR + RR without age CDF 464 has an AUC of 0.7208; and the PR+ RR+ SpO2 without age CDF 466 has an AUC of 0.7207.

It can be seen through review of FIGS. 4A & 4B that including oxygen saturation (SpO2) data type with heart rate and deterioration rate data types yields lower AUC values (both when age is and is not considered). For example, the PR + RR with age CDF 424 has an AUC of 0.7524 and the PR + RR + SpO2 with age CDF 426 has an AUC of 0.7540. The lower AUC value indicates that including blood oxygen saturation data type in the SCDS algorithm reduces discrimination performance of the algorithm. Said another way, inclusion of oxygen saturation data type reduces the performance of the SCD system below a minimum acceptable performance level. For this reason, blood oxygen saturation is not a data type that is considered by the example SCDS system for determining EDI.

The example SCDS groups pulse rate and deterioration rate in a primary set of input data. All data types within the primary set of input data are required in order for the example SCDS to provide an output having a performance greater than a minimum acceptable performance level. The example SCDS groups age into a supplemental set of input data.

The example SCDS therefore performs in two modes. In a first mode the example SCDS system considers pulse rate (PR) and deterioration rate (RR) in determining EDI. In a second mode the example SCDS system considers PR, RR, and age in determining EDI. A performance value of the first mode is estimated by AUC to be 0.7208. And, a performance value of the second mode is estimated by AUC to be 0.7542. So, the example SCDS performs better when patient age is considered. Assuming, patient age is not initially available, upon startup the example SCDS will determine EDI using the first mode (using only PR and RR). If patient age becomes available, the example SCDS will begin operating in the second mode and determine EDI using PR, RR, and age.

The methods, systems, and devices discussed above are examples. Various configurations may omit, substitute, or add various procedures or components as appropriate. For instance, in alternative configurations, the methods may be performed in an order different from that described, and that various steps may be added, omitted, or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Embodiments of the present disclosure, for example, are described above with reference to block diagrams and/or operational illustrations of methods, systems, and computer program products according to embodiments of the present disclosure. The functions/acts noted in the blocks may occur out of the order as shown in any flowchart. For example, two blocks shown in succession may in fact be executed substantially concurrent or the blocks may sometimes be executed in the reverse order, depending upon the functionality/acts involved. Additionally, or alternatively, not all of the blocks shown in any flowchart need to be performed and/or executed. For example, if a given flowchart has five blocks containing functions/acts, it may be the case that only three of the five blocks are performed and/or executed. In this example, any of the three of the five blocks may be performed and/or executed.

A statement that a value exceeds (or is more than) a first threshold value is equivalent to a statement that the value meets or exceeds a second threshold value that is slightly greater than the first threshold value, e.g., the second threshold value being one value higher than the first threshold value in the resolution of a relevant system. A statement that a value is less than (or is within) a first threshold value is equivalent to a statement that the value is less than or equal to a second threshold value that is slightly lower than the first threshold value, e.g., the second threshold value being one value lower than the first threshold value in the resolution of the relevant system.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the scope of the disclosure.

Having described several example configurations, various modifications, alternative constructions, and equivalents may be used without departing from the scope of the disclosure. For example, the above elements may be components of a larger system, wherein other rules may take precedence over or otherwise modify the application of various implementations or techniques of the present disclosure. Also, a number of steps may be undertaken before, during, or after the above elements are considered.

Having been provided with the description and illustration of the present application, one skilled in the art may envision variations, modifications, and alternate embodiments falling within the general inventive concept discussed in this application that do not depart from the scope of the following claims.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A method for providing information supportive of clinical decision making, the method comprising:
receiving (212), at a processor (110), a primary set of input data (118);
producing (214), using the processor, a first output having a first performance value based upon the primary set of input data;
receiving (216), at the processor, a signal indicating an availability of a supplemental set of input data (120);
producing (220), using the processor, a second output having a second performance value based upon the primary set of input data and the supplemental set of input data when the supplemental set of input data is available and the second performance value exceeds the first performance value.

2. The method of claim 1, further comprising:
receiving, at the processor, a second signal indicating the availability of a second supplemental set of input data; and
producing, using the processor, a third output having a third performance value based upon the primary set of input data and the second supplemental set of input data, when the second supplemental set of input data is available and the third performance value exceeds the first performance value.

3. The method of claim 1 or 2, further comprising receiving, at the processor, information concerning the contents of the primary set of input data and the supplemental set of input data.

4. The method of any of the preceding claims, further comprising providing, using the processor, a confidence value associated with the first output or the second output.

5. The method of any of the preceding claims, further comprising indicating, using the processor, an absence of the primary set of input data when the primary set of input data is at least partially incomplete.

6. The method of claim 5 wherein indicating the absence of the primary set of input data further comprises at least one of: logging the absence of the primary set of input data in an electronic medical record; mentioning the absence of the primary set of input data in a report; flagging the absence of the primary set of input data on a user interface; and providing an alarm indicating the absence of the primary set of input data.

7. The method of any of the preceding claims, further comprising storing, using the processor, at least one of the primary set of input data, the supplemental set of input data, the first output, and the second output in a log file.

8. The method of any of the preceding claims, wherein the signal indicating the availability of the supplemental set of input data is communicated by means of a publish/subscribe mechanism.

9. The method of any of the preceding claims, wherein the first output comprises a receiver operating characteristic (ROC) curve.

10. The method of any of the preceding claims, wherein the first performance value comprises at least one of: an area under the curve (AUC) calculation, and a confidence calculation.

11. A system for providing information supportive of clinical decision making, the system comprising a processor configured to carry out the method of any of claims 1-10.

12. A non-transitory computer readable medium storing instructions that are executable by a processing device, and upon such execution cause the processing device to carry out the method of any of claims 1-10.
